Europäisches Patentamt

European Patent Office

Office européen des brevets

(19)

(11) Numéro de publication: **0 049 660**
**A1**

(12) **DEMANDE DE BREVET EUROPEEN**

(21) Numéro de dépôt: **81401515.2**

(22) Date de dépôt: **30.09.81**

(51) Int. Cl.³: **A 61 F 5/47**

(30) Priorité: **03.10.80 FR 8021260**
**31.08.81 FR 8116585**

(43) Date de publication de la demande:
**14.04.82 Bulletin 82/15**

(84) Etats contractants désignés:
**AT BE CH DE GB IT LI LU NL SE**

(71) Demandeur: **LABORATOIRES CILAG-CHIMIE S.A.R.L.**
**46, rue Lauriston**
**F-75116 Paris(FR)**

(72) Inventeur: **Juif Bielsky, Marie-Christine**
**155, bd St Germain**
**F-75006 Paris(FR)**

(72) Inventeur: **Bernard, Alain Francis**
**4, rue Louise Michel**
**F-78360 Montesson(FR)**

(72) Inventeur: **Bal, Pierre J.**
**12, rue Gabrielle**
**F-75018 Paris(FR)**

(72) Inventeur: **Cattan, Serge A.**
**93, rue Jouffroy**
**F-75017 Paris(FR)**

(74) Mandataire: **Durand, Yves et al,**
**Cabinet Z. Weinstein 20, Avenue de Friedland**
**F-75008 Paris(FR)**

(54) **Nouvel appareil d'insertion pour insérer, dans l'utérus, un dispositif intra-utérin et ensemble d'un appareil d'insertion et d'un dispositif intra-utérin.**

(57) L'invention concerne un appareil et un ensemble d'insertion dans l'utérus d'un dispositif intra-utérin.

L'appareil d'insertion conforme à un premier mode de réalisation de l'invention comprend un moyen (2) formant poussoir monté coulissant dans un moyen formant guide (1) pour insérer dans la cavité utérine (50) le dispositif intra-utérin (3) et est caractérisé en ce que ledit moyen formant guide (1) comprend un corps allongé (4) de manipulation comportant un passage traversant (6) et un tube creux formant canule (8) d'insertion pour l'insertion dans la cavité utérine (50) dudit dispositif intra-utérin (3), solidaire dudit corps de manipulation et s'étendant dans le prolongement dudit corps.

L'invention permet de contrôler la position de la canule dans la cavité utérine et le positionnement correct du dispositif intra-utérin notamment.

./...

Fig:3

1

<u>Nouvel appareil d'insertion pour insérer, dans</u>
<u>l'utérus, un dispositif intra-utérin et ensemble</u>
<u>d'un appareil d'insertion et d'un dispositif</u>
<u>intra-utérin</u>

La présente invention concerne essentiellement un appareil d'insertion pour insérer, dans l'utérus, un dispositif intra-utérin et un ensemble d'un appareil d'insertion et d'un dispositif intra-utérin, utilisables dans le domaine de la contraception féminine.

On connaît déjà divers appareils d'insertion de nombreux dispositifs intra-utérins tels que par exemple un dispositif intra-utérin en forme de T permettant soit d'introduire le T contenu dans un tube inserteur, dans la cavité utérine, soit de replier aseptiquement les branches horizontales du T.

Toutefois, ces appareils connus présentent des inconvénients, et ne permettent pas de résoudre toutes les difficultés rencontrées lors de la pause de dispositifs intra-utérins.

En effet, de tels appareils soit ne maintiennent pas les branches horizontales du T en position repliée, pendant le passage du dispositif dans le canal cervical, soit demandent le repliement manuel de ces branches pour l'insertion du T dans le tube inserteur. De plus, on ne peut pas maintenir de manière certaine l'orientation du dispositif intra-utérin, et en particulier les branches horizontales du T, dans le plan frontal de la cavité utérine, plan défini par les deux trompes. De ce fait, on ne peut réaliser aisément et sûrement une mise en place correcte du dispositif intra-utérin.

Par ailleurs, tous les appareils d'insertion connus sont souvent d'une structure compliquée et la majorité d'entre eux ne permet pas d'adapter le positionnement de l'extrémité libre du tube d'insertion formant canule de l'appareil d'insertion à l'intérieur de la cavité utérine selon la profondeur de ladite cavité utérine, ce qui constitue un inconvénient sérieux pour le praticien car il est avantageux pour lui de savoir s'il se trouve à proximité du fond de l'utérus ou s'il en est très éloigné, ceci étant d'autant plus important que la profondeur de l'utérus varie grandement selon les femmes.

La présente invention a pour but de résoudre ces difficultés en proposant un appareil d'insertion d'un dispositif intra-utérin et d'un ensemble d'un appareil d'insertion et d'un dispositif intra-utérin d'une conception et d'une fabrication particulièrement simple permettant à la fois, sans intervention manuelle d'introduire aseptiquement le dispositif intra-utérin dans la cavité
utérine en le présentant et le maintenant dans le
plan frontal de la cavité utérine tout en adaptant la profondeur de l'insertion en fonction de
la profondeur de la cavité utérine.

Ce but est atteint selon la présente invention
par la réalisation d'un appareil d'insertion pour
insérer, dans l'utérus, un dispositif intra-utérin de type connu en soi, comprenant un moyen
formant poussoir monté coulissant dans un moyen
formant guide pour insérer dans la cavité utérine
ledit dispositif intra-utérin, caractérisé en ce
que ledit moyen formant guide comprend un corps
allongé de manipulation avantageusement non flexible comportant un passage traversant s'étendant
longitudinalement dans ledit corps ; et un tube
creux formant canule d'insertion d'une nature
flexible pour l'insertion dans la cavité utérine
dudit dispositif intra-utérin, solidaire dudit
corps de manipulation s'étendant dans le prolongement dudit corps de manipulation, l'orifice de
ladite canule et ledit passage du corps étant
coaxiaux pour permettre l'insertion du dispositif
intra-utérin dans l'utérus à l'aide dudit moyen
poussoir.

Selon une caractéristique de l'invention, et plus
particulièrement pour insérer un dispositif intra-utérin en forme de T, l'appareil d'insertion
selon l'invention est caractérisé en ce que le corps précité comprend deux ouvertures longitudinales diamétralement opposées, s'étendant axialement et débouchant dans le passage du corps précité, ledit passage et lesdites ouvertures étant prévus pour pouvoir recevoir ledit dispositif intra-utérin de façon que la branche du T s'étende axialement dans ledit passage longitudinal et lesdits bras dudit T s'étendent à travers lesdites ouvertures latérales. Avantageusement, ces ouvertures latérales sont débouchantes à une des extrémités du corps de manipulation précité.

Selon encore une autre caractéristiqué de l'invention, le moyen poussoir précité est formé par une tige dont une extrémité destinée à coopérer avec le dispositif intra-utérin comprend une section formant réceptacle tubulaire du dispositif intra-utérin, et un élément formant butée étant monté au voisinage de son autre extrémité.

Selon un mode de réalisation préféré de l'invention, la canule d'insertion précitée est prévue d'une longueur suffisante pour traverser sensiblement complètement la cavité utérine.

Selon une autre caractéristique intéressante de ce mode de réalisation, le passage précité du corps de manipulation et l'orifice précité de la canule ont une configuration en section transversale sensiblement circulaire, la canule d'insertion comportant de préférence un moyen formant butée coulissant externe pour engager le col de l'utérus et pour être ajusté à une position pré-

5

déterminée pour l'insertion selon la profondeur de
la cavité utérine.

De plus, la canule précitée est pourvue de graduations permettant de faciliter le positionnement de
la butée coulissante précitée en fonction de la
profondeur de la cavité utérine mesurée par le
praticien ce qui permet à ce dernier de visualiser
la profondeur d'insertion de la canule jusqu'à ce
que son extrémité libre vienne sensiblement au regard du fond de la cavité utérine.

Selon une autre caractéristique d'une importance
pratique considérable, le corps précité de manipulation est réalisé extra-plat, et est formé par
deux pièces, avantageusement identiques, de forme
généralement sensiblement plane, comportant une
partie centrale incurvée en vue de définir le passage précité après assemblage ; et un épaulement à
au moins un bords latéral pour définir lesdites
ouvertures latérales précitées.

Par ailleurs, de préférence, le moyen poussoir
comprend une longueur utile sensiblement égale ou
inférieure à la longueur totale de l'appareil
d'insertion proprement dit, ce qui permet d'éviter
que le moyen poussoir ne vienne faire saillie hors
de la canule de l'appareil d'insertion.

Ainsi, grâce à la canule selon ce mode de réalisation de l'invention , de longueur suffisante pour
traverser sensiblement complètement la cavité utérine, le praticien peut positionner sensiblement

6

l'extrémité libre de la canule au fond de la cavité utérine, cette opération étant facilitée par la présence de la butée coulissante externe disposée sur ladite canule en fonction de la profondeur de la cavité utérine préalablement mesurée par le praticien.

Ce positionnement étant obtenu, il suffit ensuite au praticien de réaliser un léger retrait, par exemple d'environ un centimètre et de faire coulisser le moyen poussoir sur toute sa longueur utile. Il n'y a donc plus à contrôler la profondeur d'insertion du moyen poussoir puisqu'on est sûr que ce dernier ne dépassera pas dans la cavité utérine.

Selon un autre mode de réalisation préféré de l'invention, l'orifice de la canule précitée a une section ovale, un moyen formant butée pour engager le col de l'utérus, en forme de collerette formant portée d'appui contre ledit col de l'utérus étant fixé à l'extrémité inférieure de ladite canule, l'orifice du corps de manipulation précité ayant une section transversale sur au moins une partie de sa longueur ajustée à la section du moyen formant poussoir.

Selon une autre caractéristique de ce mode de réalisation, les deux ouvertures latérales précitées du corps de manipulation précité sont diamétralement opposées selon un diamètre sensiblement parallèle au grand diamètre de la canule ovale précitée, et sont débouchantes à l'extrémité libre dudit corps de manipulation.

Ainsi, les branches horizontales du dispositif intra-utérin en forme de T sont repliées dans un plan contenant le grand diamètre du tube oval, ce qui permet de connaître exactement la position du dispositif intra-utérin, et de le maintenir en position correcte lors de son insertion dans la cavité utérine.

Avantageusement, pour repérer et maintenir les éléments dans la position correcte du dispositif intra-utérin lors de son insertion dans la cavité utérine, la collerette précitée présente un bord rectiligne sensiblement parallèle au grand diamètre du tube oval précité.

Par ailleurs, on peut avantageusement monter de manière coulissante un élément formant butée, tel que par exemple une bague d'arrêt ou analogue, sur le moyen formant poussoir. La position de cet élément est réglable et ajustable en fonction de la profondeur de la cavité utérine, et est repérée au moyen de graduations prévu sur ledit élément poussoir.

Ainsi, on peut insérer et poser le dispositif intra-utérin sans risque de blesser le fond de la cavité utérine.

Les modes de réalisation de l'appareil d'insertion selon l'invention présentent donc les avantages préalablement mentionnés.

D'autres caractéristiques, détails et avantages de la présente invention apparaîtront clairement au cours de la description explicative qui va suivre faite en référence aux dessins schématiques annexés illustrant deux modes de réalisation actuellement préférés de l'invention donnés uniquement à titre d'exemple et qui ne sauraient donc en aucune façon limiter la portée de la présente invention.

La figure 1 est une vue éclatée d'un premier mode de réalisation de l'appareil d'insertion selon la présente invention comportant un dispositif intra-utérin.

La figure 2 est une vue compacte représentant l'ensemble de l'appareil d'insertion de la figure 1 et du dispositif intra-utérin prêt à l'emploi.

La figure 3 est une vue schématique en coupe de l'utérus après insertion de l'appareil d'insertion de la figure 1 conforme à la présente invention, juste avant la mise en place du dispositif intra-utérin.

La figure 4 est une vue analogue à la figure 3 après un léger retrait de l'appareil d'insertion conforme au premier mode de réalisation de la présente invention pour la mise en place du dispositif intra-utérin par insertion complète du moyen poussoir.

Les figures 5 à 7 illustrent en coupe les positions successives du dispositif intra-utérin lors de son insertion dans l'appareil    d'insertion conforme à un second mode de réalisation de l'invention.

La figure 8 est une vue schématique en coupe de l'utérus avant le retrait de l'appareil d'insertion conforme au second mode de réalisation de la présente invention, après mise en place du dispositif intra-utérin.

La figure 9 est une vue de dessus en coupe suivant la ligne IX-IX de la figure 5.

La figure 10 est une vue de dessus suivant la flèche X de la figure 7. Et

La figure 11 est une vue de côté suivant la flèche XI de la figure 5.

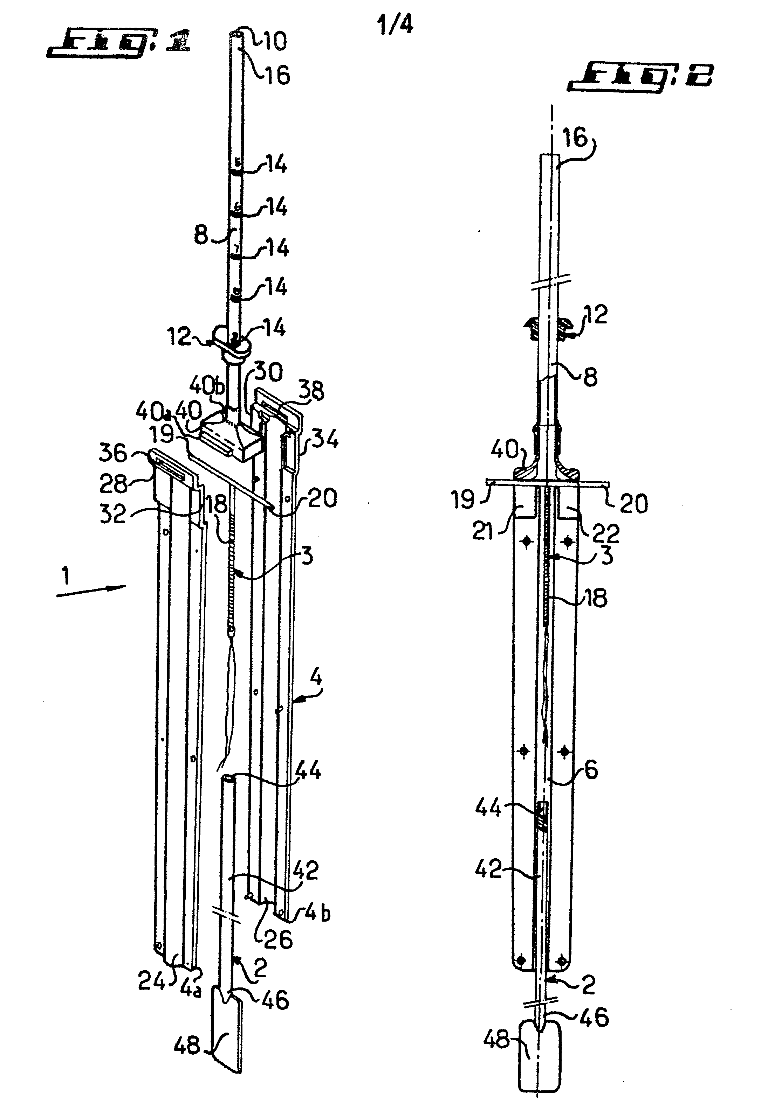

Suivant un premier mode de réalisation préféré de la présente invention représenté aux figures 1 à 4 et en se reportant plus particulièrement à la figure 1, un appareil d'insertion conforme à la présente invention comprend un moyen formant guide 1 et un moyen formant poussoir 2 monté coulissant dans le moyen formant guide 1 pour insérer dans la cavité utérine le dispositif intra-utérin 3.

Le moyen formant guide 1 comprend un corps allongé 4 de manipulation avantageusement non flexible comportant un passage traversant 6 s'étendant longitudinalement dans ledit corps 4. L'appareil d'insertion selon l'invention comprend en outre un tube creux formant canule 8 d'insertion d'une nature flexible pour l'insertion dans la cavité utérine dudit dispositif intra-utérin 3. La canule d'insertion 8 est solidaire du corps de manipulation 4 et s'étend dans le prolongement du corps 4. On notera que l'orifice 10 de la canule 8 et le passage 6 du corps 4 sont coaxiaux pour permettre l'insertion du dispositif intra-utérin dans l'utérus à l'aide du moyen poussoir 2.

Par ailleurs, également selon l'invention la canule d'insertion 8 est prévue d'une longueur suffisante pour traverser sensiblement complètement la cavité utérine.

Selon un mode de réalisation particulier, le passage 6 du corps de manipulation 4 et l'orifice 10 de la canule 8 ont une configuration en section transversale sensiblement circulaire. En outre, de préférence la canule 8 d'insertion comporte un moyen 12 formant butée coulissante externe pour engager le col de l'utérus et pouvant être ajusté à une position prédéterminée pour l'insertion selon la profondeur de la cavité utérine. Ce positionnement est avantageusement facilité par la provision de la canule avec des graduations 14 par exemple indiquant la longueur d'insertion de la canule depuis son extrémité libre 16.

Ce moyen formant butée 12 peut comporter un bord aplati afin de le faire correspondre au plan du corps 4 bien que cette disposition ne soit pas indispensable tandis que pour améliorer le grippage du moyen formant butée 12 sur la canule 8
l'orifice du moyen formant butée 12 qui est généralement formé par une bague peut comporter
des saillies axiales parallèles à l'axe de
l'orifice de la bague constituant le moyen formant butée 12 afin d'en diminuer son diamètre et
d'offrir une résistance au coulissement relativement à la canule 8.

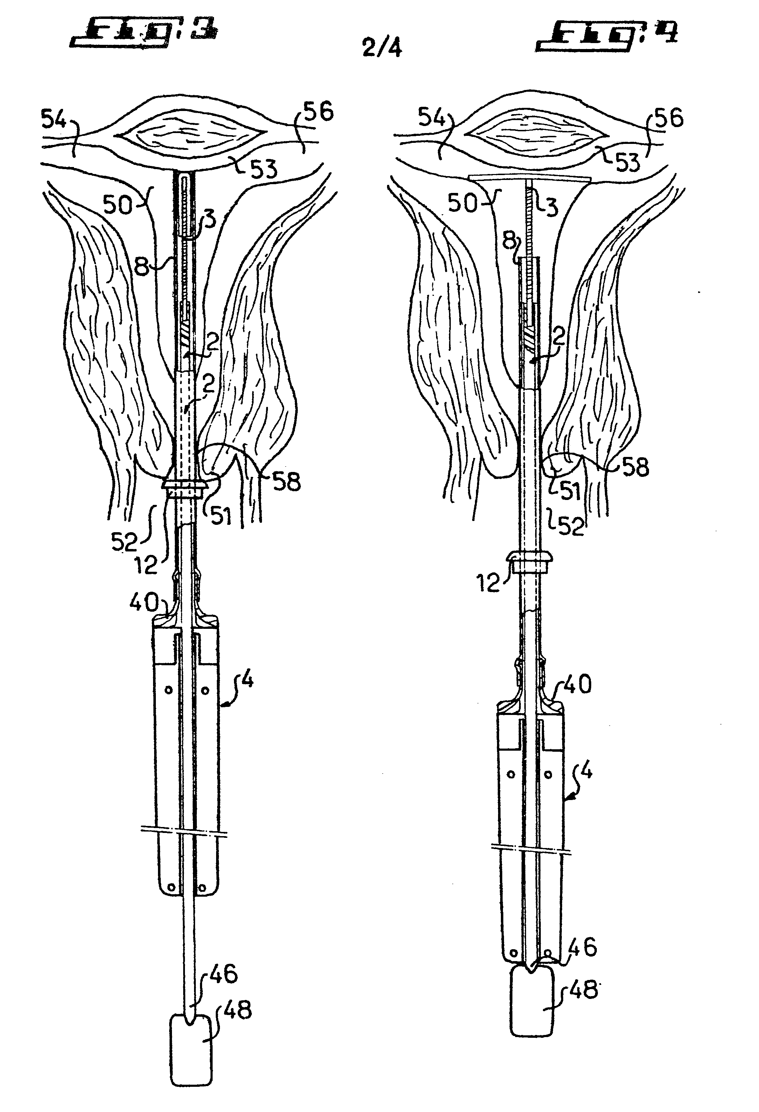

Dans le cas d'un dispositif intra-utérin 3 ayant
une branche 18 et au moins un bras flexible 19,
20 s'étendant normalement sensiblement horizontalement, l'appareil d'insertion de la présente
invention est en outre caractérisé en ce que le
corps 4 de manipulation comprend au moins une
ouverture latérale correspondante 21, 22, respectivement s'étendant axialement et débouchant dans
le passage traversant 6 du corps 4. On peut voir
ainsi que le passage 6 et l'ouverture latérale 21,
22 sont prévus pour pouvoir recevoir le dispositif
intra-utérin 3, comme représenté (figure 2) de
façon que la branche 18 s'étend axialement dans le
passage traversant longitudinal 6 et que le bras
19, 20 s'étende à travers l'ouverture latérale 21,
22 respectivement.

Avantageusement, le corps 4 de manipulation est
réalisé plat ou même extra-plat et est formé par
deux pièces 4a, 4b avantageusement identiques de
forme générale sensiblement plane comportant une
partie centrale incurvée 24, 26 en vue de définir
le passage traversant 6 après assemblage ; et un
épaulement 28, 30 ; 32, 34 à au moins un bords
latéral pour définir l'ouverture latérale précitée 21, 22, respectivement.

On notera que, dans l'exemple représenté comme on peut le voir à partir de la figure 1, de préférence la canule d'insertion 8 n'est pas directement montée sur le corps 4 mais sur une tête intermédiaire 40 elle-même emprisonnée entre les deux pièces 4a, 4b complémentaires constituant le corps 4, par exemple à l'aide d'épaulements 40a, 40b et d'encoches 36, 38.

Ainsi, l'appareil d'insertion est essentiellement réalisé en quatre pièces qui sont de préférence réalisées en matière plastique, solidarisées entre elles de manière habituelle, et de préférence par soudage notamment aux ultrasons.

Cette réalisation permet une automatisation de la fabrication de l'appareil d'insertion au cours de laquelle avant assemblage des pièces 4a, 4b on peut disposer automatiquement le dispositif intra-utérin 3, entre lesdites pièces 4a, 4b de manière à obtenir un ensemble formé par l'appareil d'insertion et le dispositif intra-utérin susceptible d'être aisément aseptisé avant d'être utilisé par le praticien, et sans intervention manuelle pour le positionnement du dispositif intra-utérin dans l'appareil d'insertion.

Selon une autre caractéristique de l'invention, le moyen formant poussoir 2 est formé par une tige 42 dont une extrémité 44 est destinée à coopérer avec le dispositif intra-utérin 3 et comporte une section formant réceptacle tubulaire au dispositif intra-utérin. Avantageusement, l'autre extrémité 46 comporte un élément 48 formant butée
qui de préférence forme corps avec la tige 42,
cet élément formant butée étant réalisé dans
l'exemple représenté, de manière extra-plate pour
faciliter la manipulation par le praticien en
vue de son insertion dans le passage traversant
6 du corps 4 de l'appareil d'insertion.

On notera ainsi que le dispositif intra-utérin 3
présente ses bras 19, 20 dans le plan général
des pièces 4a, 4b formant le corps 4 de l'appareil d'insertion. Ainsi, le corps de manipulation
4 permet au praticien de positionner aisément le
dispositif intra-utérin dans le plan frontal de
la cavité utérine, définie par les deux trompes.

On décrira maintenant les opérations nécessaires
pour la pose du dispositif intra-utérin 3 dans
une cavité utérine 50 en utilisant l'appareil
d'insertion selon la présente invention, bien que
ces opérations soient aisément compréhensibles
pour un homme de l'art.

Les différentes étapes de l'insertion de là pause
du dispositif intra-utérin 3 sont représentées
aux figures 2 à 4.

Comme on l'a noté précédemment, tout d'abord le
praticien a déterminé quelle était la profondeur
de la cavité utérine et a ensuite positionné le
moyen formant butée coulissante 12 à la graduation correspondant à la profondeur mesurée de la
cavité utérine. Ainsi, on peut donc constater que

14

la longueur du tube creux 15 formant canule est
normalement prévue suffisante pour être supérieure à la profondeur de toutes les cavités
utérines que l'on peut trouver dans la pratique.
Ceci constitue l'une des caractéristiques essentielles de la présente invention.

Ainsi, après positionnement du moyen 12 formant
butée coulissante, l'appareil d'insertion se
trouve dans la position représentée à la figure
2. On notera que dans cette position le dispositif intra-utérin 3 est dans sa position de départ
avec les bras 19, 20 passant au travers des ouvertures latérales 21, 22 du corps 4 de sorte que
ledit bras 19, 20 se trouve en position sensiblement horizontale.

A partir de cette position représentée à la figure 2, le praticien dispose l'extrémité libre 44
du moyen poussoir 2 dans le passage traversant 6
du corps 4 et réalise une poussée en avant sur le
pied 18 du dispositif intra-utérin 3, afin de
l'introduire dans la canule 8 après repliement
des bras flexibles 19, 20 le long du pied 18. Le
praticien continue cette poussée à l'aide du
moyen poussoir jusqu'à ce que la tête avant du
dispositif intra-utérin 3 sensiblement affleure
l'extrémité libre 16 dans l'orifice 10 de la canule 14.

A partir de cette nouvelle position, le praticien
insère l'appareil d'insertion dans le vagin 52 de
telle manière que le moyen formant butée 12 vienne en butée contre le col utérin 51, position pour laquelle l'extrémité libre 16 de la canule se trouve également sensiblement en contact contre le fond 53 de la cavité utérine 50.

Bien entendu, le praticien peut calculer le positionnement du moyen formant butée 12, de sorte que l'extrémité libre 16 de la canule 14 ne touche pas réellement le fond 53 de la cavité utérine 50.

Cette longueur de la canule 14 permet donc au praticien d'être sûr que l'extrémité libre 16 de la canule se trouve bien à proximité du fond de la cavité utérin quelle que soit la profondeur de la cavité utérine de ses patientes.

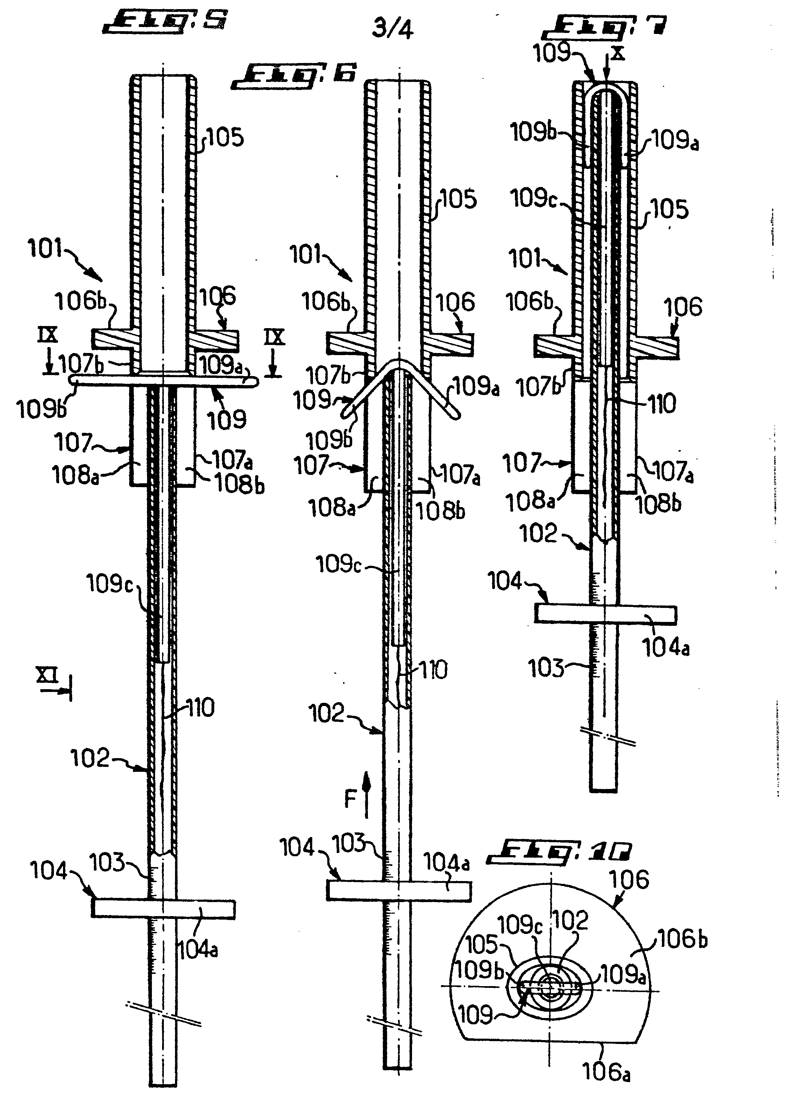

Cette position est représentée à la figure 3.

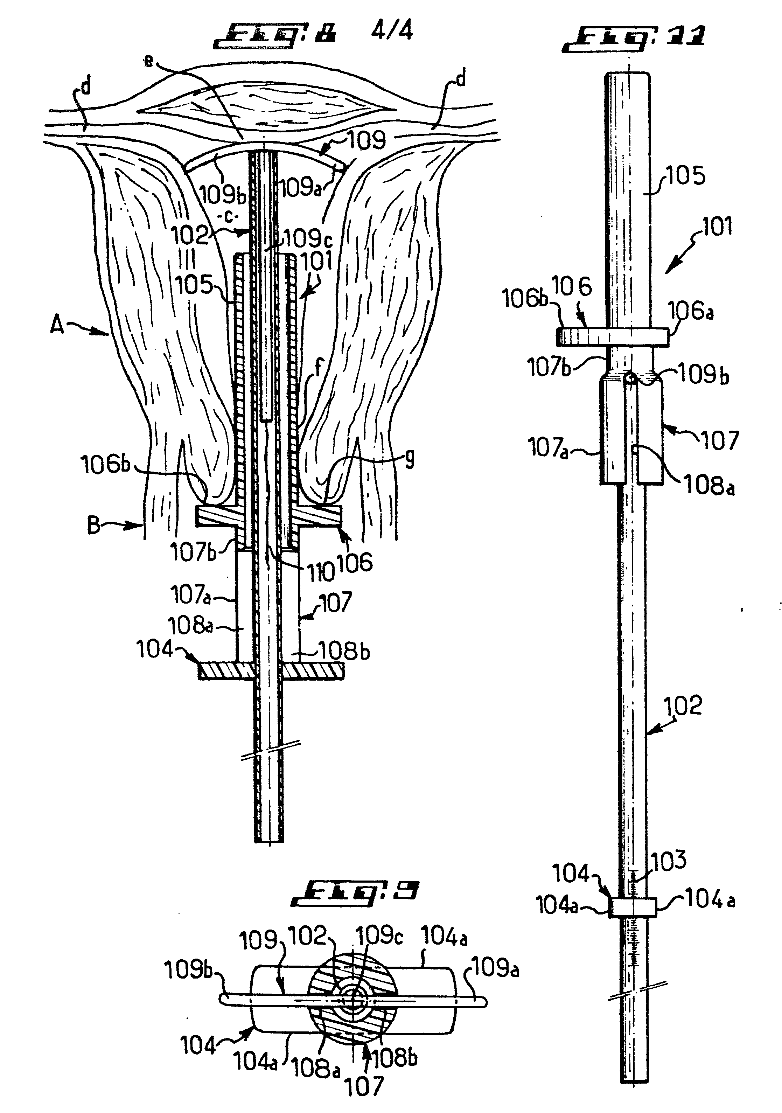

A partir de cette position, le praticien réalise un léger retrait par exemple d'environ 1 cm, et actionne alors à nouveau le moyen poussoir 2, jusqu'à ce que l'élément formant butée 48 vienne en butée contre le bord du corps 4, comme représenté à la figure 4. Ce mouvement du moyen poussoir 2 a permis de faire sortir dans la cavité utérine 50 le dispositif intra-utérin 3 dont les bras 19, 20 se sont ouverts comme représenté à la figure 4.

L'opération de pose se termine alors par le retrait commun du corps 4 et du moyen poussoir 2 comme cela se comprend aisément pour un homme du métier.

On notera que avec ce mode de réalisation l'appareil d'insertion selon la présente invention, les bras du dispositif intra-utérin sont toujours essentiellement disposés dans le plan défini par la forme extra-plate du corps 4. Ainsi, le praticien peut déterminer le positionnement du dispositif intra-utérin dans le plan des trompes 54, 56 (comme on le voit à la figure 4) à l'aide du positionnement du plan défini par le corps 4 de l'appareil d'insertion, le corps 4 étant facilement visible pour le praticien. En outre, comme la longueur du moyen poussoir 2 est calculée pour ne pas être supérieure à la longueur totale du corps 4 et de la canule 8, le praticien n'a pas besoin de calculer la profondeur de pénétration du moyen poussoir dans le corps 4 en fonction de la profondeur de la cavité utérine mais au contraire à chaque fois exerce une poussée sur le moyen poussoir jusqu'à ce que l'élément d'arrêt 48 vienne en butée contre le bord du corps 4.

Selon un second mode de réalisation de la présente invention et en se reportant à la figure 5, un appareil d'insertion comprend à sa partie supérieure un moyen formant guide 101 ou tube-guide, et un moyen formant poussoir 102 ou tube-poussoir monté coulissant dans le tube-guide 101.

Le tube-poussoir 102 est un tube cylindrique dont la surface externe est graduée.

Une bague d'arrêt 104 comprenant au moins un bord rectiligne 104a, est montée coulissante sur le tube-poussoir 102.

Cette bague est positionnée sur une graduation 103 du tube-poussoir 102 déterminée en fonction de la profondeur de la cavité utérine.

Selon l'invention, le moyen formant guide 101 ou tube-guide comprend à sa partie supérieure un tube 105 ou canule de section ovale. L'extrémité inférieure de la canule 105 comprend une collerette 106 définissant une portion de disque. Le bord rectiligne 106a de la collerette 106 est sensiblement parallèle au grand diamètre 105a de la canule 105.

Le tube-guide 101, conforme à l'invention, comprend également un second tube 107 ou corps de manipulation fixé à la partie inférieure 106b de la collerette et axialement aligné avec la canule 105. Comme on le voit sur les figures 9 et 11, ce corps 107 comprend deux ouvertures 108a et 108b s'étendant longitudinalement depuis l'extrémité inférieure du tube 107 sur une partie 107a de ce tube. Ces deux ouvertures 108a et 108b sont diamétralement opposées selon un diamètre sensiblement parallèle au grand diamètre 105a de la canule 105.

Selon une réalisation préférée de l'invention, la partie 107a du corps 107 comprenant les ouvertures 108a et 108b, a un diamètre ajusté au diamètre extérieur du tube-poussoir 102, tandis que la partie supérieure 107b du corps 107 a avantageusement une section ovale.

Comme on le voit sur la figure 5, le dispositif
intra-utérin 109 en forme de T ou appareil contraceptif, comprend des bras horizontaux 109a
et 109b et une branche verticale 109c à l'extrémité inférieure de laquelle est fixé un fil
souple 110. Ce dispositif intra-utérin est réalisé de préférence en une matière souple et
élastique.

En se référant aux figures 5, 9 et 11, on voit
que la branche verticale 109c du dispositif intra-utérin 109 est engagée dans le tube-poussoir
102 et que les branches horizontales 109a et
109b sont reçues dans les ouvertures 108a et
108b du corps 107. En outre, les figures 9 et 11
montrent que les bras horizontaux sont maintenus
parallèles au bord rectiligne 106a de la collerette 106 et au bord rectiligne 104a de la
bague d'arrêt 104.

On décrira maintenant les opérations nécessaires
pour la pose du dispositif intra-utérin 109 dans
une cavité utérine c en utilisant le dispositif
d'insertion de la présente invention.

Les différentes étapes de l'insertion et de la
pose du dispositif intra-utérin 109 sont représentées sur les figures 5 à 8.

La figure 5 illustre l'ensemble appareil d'insertion, dispositif intra-utérin avant son utilisation. On voit que le dispositif intra-utérin 109
est engagé dans l'extrémité supérieure du tube-poussoir 102 et dans les ouvertures 108a et 108b
du corps de manipulation 107. Les bras horizontaux 109a et 109b du dispositif intra-utérin sont
en butée contre le fond arrondi des ouvertures
108a et 108b.

Il est aisé de comprendre que le positionnement
du dispositif intra-utérin 109 dans l'appareil
d'insertion de l'invention est réalisé de manière
aseptique.

Ensuite on positionne la bague d'arrêt 104 sur
une graduation 103 du tube-poussoir 102, en fonction de la profondeur de la cavité utérine $\underline{c}$.

Dans une deuxième étape, illustrée par la figure
6, on applique une poussée F sur le tube-poussoir
102 afin d'insérer le dispositif intra-utérin
dans la canule 105. Cette poussée du tube-poussoir 102 provoque le repliage des branches horizontales 109a et 109b du dispositif intra-utérin
109 vers les parois du tube-poussoir 102. Il
suffit alors de continuer d'appliquer une poussée
F sur le tube-poussoir de manière à ce que l'extrémité supérieure du dispositif intra-utérin 109
affleure l'ouverture supérieure de la canule 105,
comme cela est illustré sur la figure 7. Comme on
le voit sur la figure 10, le dispositif d'insertion objet de l'invention permet de maintenir en
position bloquée les bras 109a et 109b dans le
plan contenant le grand diamètre 105a de la canule 105. D'autre part, les bras 109a et 109b du
dispositif intra-utérin 109 repliés contre la paroi du tube-poussoir empêchent ce dernier de tourner dans la canule 105. En outre, on voit clairement que les branches horizontales 109a et 109b sont dans un plan parallèle au plan contenant l'arête 106a rectiligne de la collerette 106, ainsi il sera aisé de positionner correctement le dispositif intra-utérin dans la cavité utérine c.

En se référant maintenant à la figure 8 on va décrire l'étape finale de la pose du dispositif intra-utérin 109 dans l'utérus A.

L'ensemble représenté à la figure 7 est introduit dans le vagin B de telle manière que la collerette 106 vienne en butée contre le col utérin g. La canule 105 a alors pénétré dans le canal cervical f et son extrémité supérieure débouche dans la cavité utérine c.

L'appareil d'insertion de la présente invention permet, par le contrôle et l'ajustement de la position des bords rectilignes 106a, 104a de la collerette 106 et de la bague d'arrêt 104, d'ajuster et de maintenir le dispositif intra-utérin dans le plan frontal défini par les deux trompes d.

Pour réaliser sa mise en place finale, il suffit de pousser le tube-poussoir 102 jusqu'à ce que la bague d'arrêt 104 vienne en butée contre l'extrémité inférieure du corps 107, pour libérer les bras horizontaux 109a, 109b du dispositif intra-utérin 109. Ces bras horizontaux 109a et 109b viennent en contact avec le bord de la cavité utérine c au niveau de l'ouverture des trompes d.
Il suffit alors de retirer l'appareil    d'insertion de l'invention pour terminer la pose du dispositif intra-utérin 109.

Il est bien entendu que le moyen formant guide de
ce second mode de réalisation peut être composé de
plusieurs éléments assemblés ou être une unité
unique obtenue par moulage ou usinage, sans pour
cela sortir du cadre de l'invention.

On notera par ailleurs comme mentionné précédemment que l'invention permet d'obtenir le positionnement du dispositif intra-utérin dans la cavité utérine 50 sans toucher au dispositif intra-
utérin ce qui permet de réaliser une opération
totalement aseptique tout en obtenant un positionnement correct et aisé du dispositif intra-
utérin dans la cavité utérine. On notera également que l'invention permet une insertion du dispositif intra-utérin sans contact avec les parois
du canal cervical.

Bien entendu, l'invention n'est nullement limitée
aux modes de réalisation décrits et représentés
qui n'ont été donnés qu'à titre d'exemple. Ainsi,
l'invention comprend toutes les variantes d'exécution notamment concernant les matériaux utilisés
pour réaliser le corps 4, 107 et la canule 8, 105.
Généralement, le corps 4, 107 est réalisé en polystyrène cristal alors que la canule 8, 105 qui
doit être flexible est réalisée en polyéthylène.

L'invention comprend donc tous les moyens constituants des équivalents techniques des moyens décrits, ainsi que leurs combinaisons exécutées dans l'esprit de la présente invention.

Revendications

1. Appareil d'insertion pour insérer, dans l'utérus, un dispositif intra-utérin de type connu en soi, du type comprenant un moyen formant poussoir monté coulissant dans un moyen formant guide pour insérer dans la cavité utérine ledit dispositif intra-utérin, caractérisé en ce que ledit moyen formant guide comprend un corps allongé de manipulation avantageusement non flexible comportant un passage traversant s'étendant longitudinalement dans ledit corps, et un tube creux formant canule d'insertion d'une nature flexible pour l'insertion dans la cavité utérine dudit dispositif intra-utérin, solidaire dudit corps de manipulation et s'étendant dans le prolongement dudit corps, l'orifice de ladite canule et ledit passage dudit corps étant coaxiaux pour permettre l'insertion du dispositif intra-utérin dans l'utérus à l'aide dudit moyen poussoir.

2. Appareil selon la revendication 1, pour dispositif intra-utérin en forme de T, caractérisé en ce que le corps précité comprend deux ouvertures longitudinales diamétralement opposées, s'étendant axialement et débouchant dans le passage du corps précité, ledit passage et lesdites ouvertures étant prévus pour pouvoir recevoir ledit dispositif intra-utérin de façon que les branches du T s'étendent axialement dans ledit passage longitudinal et que lesdits bras dudit T s'étendent à travers lesdites ouvertures latérales.

3. Appareil selon la revendication 2, caractérisé
en ce que les ouvertures latérales précitées sont
débouchantes à une des extrémités du corps de manipulation précité.

4. Appareil d'insertion selon l'une quelconque des
revendications précédentes, caractérisé en ce que
le moyen poussoir précité est formé par une tige
dont une extrémité destinée à coopérer avec le
dispositif intra-utérin comprend une section formant réceptacle tubulaire du dispositif intra-utérin, un élément formant butée étant monté au voisinage de son autre extrémité.

5. Appareil d'insertion selon l'une des revendications précédentes, caractérisé en ce que la canule
d'insertion précitée est prévue d'une longueur
suffisante pour traverser sensiblement complètement
la cavité utérine.

6. Appareil d'insertion selon l'une des revendications précédentes, caractérisé en ce que le passage
précité du corps de manipulation et l'orifice précité de la canule ont une configuration en section
transversale sensiblement circulaire ; la canule
d'insertion comportant de préférence un moyen formant butée coulissante externe pour engager le col
de l'utérus et pouvant être ajusté à une position
prédéterminée pour l'insertion selon la profondeur
de la cavité utérine.

7. Appareil d'insertion selon l'une des revendications précédentes, caractérisé en ce que la canule
précitée est pourvue de graduations.

8. Appareil d'insertion selon l'une quelconque des revendications précédentes, caractérisé en ce que l'élément formant butée du moyen poussoir précité est fixé à l'extrémité de la tige précitée, et de préférence fait corps avec ladite tige.

9. Appareil d'insertion selon l'une quelconque des revendications précédentes, caractérisé en ce que le corps précité est de forme sensiblement plane, de préférence extra-plane, et est de préférence formé par deux pièces avantageusement identiques de forme généralement plane comportant une partie centrale incurvée en vue de définir le passage précité après assemblage ; et comportant un épaulement à au moins un bords latéral pour définir les ouvertures latérales précitées.

10. Appareil d'insertion selon la revendication 9, caractérisé en ce que la canule d'insertion précitée est montée sur une tête elle-même emprisonnée entre les deux pièces identiques et complémentaires précitées.

11. Appareil d'insertion selon l'une quelconque des revendications précédentes, caractérisé en ce que le moyen poussoir a une longueur utile sensible égale ou inférieure à la longueur totale de l'appareil d'insertion proprement dit.

12. Appareil d'insertion selon l'une des revendications 1 à 4, caractérisé en ce que l'orifice de la canule précitée a une section ovale, un moyen formant butée pour engager le col de l'utérus, en forme de collerette formant portée d'appui contre ledit col de l'utérus étant fixé à l'extrémité inférieure de ladite canule, l'orifice du corps de manipulation précité ayant une section transversale sur au moins une partie de sa longueur ajustée à la section du moyen formant poussoir.

13. Appareil d'insertion selon la revendication 12, caractérisé en ce que les deux ouvertures latérales précitées du corps de manipulation précité sont diamétralement opposées selon un diamètre sensiblement parallèle au grand diamètre de la canule ovale précitée, et sont débouchantes à l'extrémité libre dudit corps de manipulation.

14. Appareil d'insertion selon la revendication 12 ou 13, caractérisé en ce que la collerette précitée présente un bord rectiligne sensiblement parallèle au grand diamètre de la canule ovale précitée.

15. Appareil selon l'une des revendications 12 à 14, caractérisé en ce que le moyen poussoir précité est pourvu de graduations, et l'élément formant butée précité dudit moyen poussoir, tel que par exemple une bague d'arrêt, est monté coulissant et réglable sur ledit moyen poussoir.

16. Appareil selon la revendication 15, caractérisé en ce que la bague d'arrêt précitée comprend un bord rectiligne.

17. Ensemble d'insertion, caractérisé en ce qu'il comprend un dispositif intra-utérin flexible et un appareil d'insertion tel que défini selon l'une des revendications 1 à 16.

1/4

Fig. 1   Fig. 2

Fig. 3

Fig. 4

Fig. 5

Fig. 6

Fig. 7

Fig. 10

Fig. 8  4/4

Fig. 9

Fig. 11

Office européen **RAPPORT DE RECHERCHE EUROPEENNE**
des brevets

Numéro de la demande
EP 81 40 1515

## DOCUMENTS CONSIDERES COMME PERTINENTS

| Catégorie | Citation du document avec indication, en cas de besoin, des parties pertinentes | Revendication concernée |
|---|---|---|
| X | <u>FR - A - 2 385 410</u> (ORTHO PHARMA-CEUTICAL CORP.)<br><br>* page 8, ligne 23 à page 11, ligne 17; page 15, lignes 29-38; revendication 6; figures 1-10 * | 1-4,6-9,11-13,17 |
| | <u>US - A - 4 026 281</u> (MAYBERRY)<br><br>* colonne 2, lignes 11-60; colonne 3, lignes 23-37; figures 1,8-10 * | 2,4,6,15 |

**CLASSEMENT DE LA DEMANDE (Int. Cl. 3)**

A 61 F 5/47

**DOMAINES TECHNIQUES RECHERCHES (Int. Cl. 3)**

A 61 F

**CATEGORIE DES DOCUMENTS CITES**

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 07.01.1982 | BARTLETT |

OEB Form 1503.1 06.78